# EUROPEAN PATENT APPLICATION

(11) **EP 4 585 236 A1**
(43) Date of publication of application: **16.07.2025**
(21) Application number: 24151643.4
(22) Date of filing: 12.01.2024
(51) Int. Cl.: A61M 1/14, A61M 1/28

(54) **SYSTEM WITH ORGANIZER FOR MEDICAL TREATMENT, AND METHOD**

(71) Applicant: Fresenius Medical Care Deutschland GmbH, 61352 Bad Homburg (DE)
(72) Inventor: Jonas, Joerg, 61352 Bad Homburg (DE); Chamney, Paul, 61352 Bad Homburg (DE); Weber, Tobias, 61352 Bad Homburg (DE)
(74) Representative: Bobbert & Partner Patentanwälte PartmbB

(57) **Abstract**

The present invention relates to a system comprising an organizer (1) for medical treatment, preferably for a dialysis treatment of a patient (P), in particular for a peritoneal dialysis treatment. The organizer (1) comprises a first reusable unit (10), comprising at least one first connection site (11) for connecting it to a second disposable unit (20), the second disposable unit (20) being or comprising lines.

## Description

The present invention relates to a system according to claim 1, a method according to claim 14 and a second disposable unit according to claim 15 or according to the preambles or generic terms of each of these claims.

From DE 10 2018 113 322 organizers for peritoneal dialysis are known.

An object of the present invention is to suggest a system comprising another organizer for medical treatment.

Moreover, a method for using the same should be suggested.

The objective of the present invention is achieved by the system having the features of claim 1. It is further achieved by the method having the features of claim 14 and/or by the second disposable unit having the features of claim 15.

According to the present invention, a system comprising the features of claim 1 is suggested.

The system comprises an organizer for medical treatment, preferably for a dialysis treatment of a patient, in particular, for a peritoneal dialysis treatment.

The organizer comprises a first reusable unit. The first reusable unit comprises at least one first connection site for connecting it to a second disposable unit, one that is usually disposed after use. The second disposable unit to be connected is of a type that consists of or comprises lines, intended for connecting the two or more liquid bags with the patient in fluid communication.

According to the present invention, there is suggested a method for preparing an organizer before treating a patient by dialysis encompassing the features of claim 14.

The method encompasses the steps of providing a system according to the present invention and to break a frangible or the like comprised by the second disposable unit in order to establish or allow a fluid communication through the part of the system comprising the frangible, e.g. between the two or more liquid bags and the patient once connected to the second disposable unit.

The herein discussed system is subject-matter of the present invention and, therefore, according to the present invention. The herein discussed method is subject-matter of the present invention and, therefore, according to the present invention.

The herein discussed disposable unit, addressed as "second" disposable unit, is also subject or aspect of the present invention. It may be embodied as in any embodiment of the system or any other disclosure made herein.

Embodiments according to the present invention may comprise one or several of the features mentioned supra and/or in the following based on any of the independent claims. Thereby, the herein mentioned features may be the subject-matter in any combination based on any of the independent claims, unless the person skilled in the art considers the particular combination to be technically impossible.

Advantageous developments of the present invention are each subject-matter of the dependent claims and embodiments.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" (or "a/an") as encompassing "at least one". This understanding is also equally encompassed by the present invention as the interpretation that a numerical word, for example, "one" (or "a/an") may alternatively mean "exactly one", wherever this is evidently technically possible in the view of the person skilled in the art. Both of these understandings are encompassed by the present invention and apply herein to all used numerical words.

In case of doubt, the person skilled in the art will understand spatial information like "above", "below", "upper", "lower", "left" or "right" whenever they are herein mentioned, as a spatial indication with reference to the alignment in the figures appended hereto. "Bottom" is closer to the bottom of the figure than "top".

Whenever an embodiment is mentioned herein, it represents an exemplary embodiment according to the present invention.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

When method steps are mentioned herein, then the optional control device of the system is in several embodiments configured in order to execute, in any combination, one or several of these method steps, in particular when these are automatically executable steps, or in order to accordingly control corresponding devices which preferably correspond with their names to the designation of the respective method step (for example, "determining" as a method step and "apparatus for determining" for the apparatus) and which may likewise be part of the apparatus(es) according to the present invention or connected thereto in signal communication.

When "programmed" or "configured" is mentioned herein, then these terms may in some embodiments be interchangeable.

When a signal communication or communication connection between two components is mentioned herein, this may be understood to mean a connection that exists during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

When method steps are mentioned herein, then it is provided in several embodiments that these steps are carried out prior to or after a treatment of the patient, for instance during the time when the patient is not connected, e.g. via the third (patient) line or the like, to the apparatus or that a treatment has not begun yet or is already completed.

The control device, that may be a control device or a closed-loop control device may prompt the execution of all or substantially all of the method steps.

Whenever the term "line" is mentioned herein, these lines are suitable and/or provided for guiding fluids or liquids.

Whenever the term "automatic line clamp" is mentioned herein, this may include automated line clamps, electromagnetic line clamps, and the like.

Whenever the term "sealing pin" is mentioned herein, this may be synonymous to "safety pin" or "securing pin". In some embodiments these terms may be interchangeable.

The "third line" may herein also include a "patient line" which may in several embodiments be realized as a catheter extension.

The terms "sensor", "reader" and/or "scanner" may be interchangeable in some embodiments.

In certain embodiments, the system comprises a dialysis liquid source or bag and/or a drain or drain bag. In others, it does not.

In some embodiments, the system according to the present invention comprises a power source or is connected to such, e. g., a battery, rechargeable battery, accumulator or the like.

In several embodiments, the first reusable unit is or comprises a tray. Optionally, the first reusable unit does, before being connected to the second disposable unit, not comprise lines for guiding liquids to or from the patient.

In several embodiments, the first reusable unit comprises or is connected to the second disposable unit. The second disposable unit may be connected to the first reusable unit using the first connection site, and/or vice versa.

For this purpose, the first connection site may be or comprise a at least one first reception site, for example a groove and/or a clamping section. This applies for any connection site provided at or on the first reusable unit. Further, any suitable number of connection sites at or on the first reusable unit is encompassed in the present invention.

In some embodiments, the second unit comprises a first line for guiding fresh dialysis liquid from a dialysis liquid source or bag (optionally comprised by the system as well) and a second line for guiding used dialysis liquid, herein also referred to as dialysate, (in)to a drain or drain bag (optionally comprised by the system as well). Both lines are connected to each other, i.e. are in fluid connection, preferably via a Y-piece, connector, or another line section. The Y-piece or the other line section are in fluid connection or connected to a third line for guiding fresh dialysis liquid from or through the organizer or the second disposable unit thereof, towards and used dialysis liquid away from the patient to or through the organizer or the second disposable unit thereof.

In several embodiments, the second disposable unit comprises a first line for guiding fresh dialysis liquid from a dialysis liquid source or bag towards and for guiding used dialysis liquid to a drain or drain bag away from the organizer and a third line that is in fluid connection or connected to the first line. The third line serves for guiding fresh dialysis liquid towards and used dialysis liquid away from the patient. The second line mentioned in the preceding paragraph is optionally not present.

In several embodiments, the first line comprises an, in particular manual, line clamp.

In some embodiments, the second line comprises an, in particular manual, line clamp.

In several embodiments, the third line comprises an, in particular manual, patient line clamp.

In some embodiments, the Y-piece or the third line comprise or are embodied as a measurement chamber or measurement area. The measurement chamber or area may differ from adjacent sections of the Y-piece or the third line in structure, opaqueness, wall thickness, and the like.

In several embodiments, the Y-piece, the third line or the measurement chamber comprises a manipulation limit or rim. A manipulation limit or rim may be a protrusion that hinders the second disposable unit to slip through the user's finger while setting up the system. Thus, it may protect the measurement chamber or area from being touched or contaminated inadvertently by the user. The correctness and precision of future measurements may, thus, be ensured.

In several embodiments , the third line, that may comprise or be elongated by a patient line and that may also comprise a patient line connector (short: patient connector) for connecting the patient line to other parts of the third line, e.g., to the measurement chamber or measurement area, comprises a connection site or coupling site, preferably a screw fitting, for connecting parts of the third line with a sealing pin that, in turn, may also be part of the third line or comprised therein. The connection site may be, or may be part of, the patient line connector, if provided.

In some embodiments, a part of the third line connected to the optional Y-piece, the measurement chamber or measurement area or any other part of the third line has a first connector part, while the patient connector has a second connector part, connectable to the first connector part. The first and the second connector part can fit into each other or complement each other, e.g. as screw fitting and screw, preferably in order to establish a fluid-tight connection. Both connector parts feature a lumen for conducting liquid through them. Both connector parts may be arranged to be connected to the sealing pin mentioned herein. Both connector parts may be arranged to form a common lumen for housing the sealing pin mentioned herein.

In several embodiments, the sealing pin, or a part thereof in turn provides a lumen itself through which fluid may flow in the longitudinal direction of the sealing pin. The sealing pin may be arranged, at least in part, in the lumen of the first connector part, the second connector part or the common lumen so that liquid can flow through and/or around the sealing pin and, hence, from a line connected to the first connector part to a line connected the second connector part, or vice versa.

In some embodiments, the sealing pin comprises at its outer side a threaded portion that is adapted to enter into threaded engagement with the first connector part that may, in turn, comprise a threaded portion on its inside.

In several embodiments, the sealing pin furthermore has a first bounding element. The first bounding element may be an element restricting the movement of the sealing pin relative to the second connector part, or it may be part of a mechanism therefor. The first bounding element may be a rim or, preferably, has the form of teeth, of a toothed block, of protrusions, or the like that may restrict a rotation of the sealing pin in at least one rotation direction if in contact with a second bounding element of or another part of the second connector part suitable for engaging with the first bounding element.

In some embodiments, the sealing pin preferably has a fixing element at its end opposite the first bounding element, by which fixing element the sealing pin can be releasably or non-releasably fixed to a closure element, preferably via a latched connection and/or via an undercut, such as a sealing or disinfection cap.

In several embodiments, the second connector part comprises a receiving section, preferably concentrically surrounding the lumen of the second connector part, for receiving a first threaded section arranged at the outer side of the first connector part, wherein the receiving section of the second connector part has a threaded portion on its inner side that is adapted to enter into threaded engagement with the first threaded section of the first connector part.

In some embodiments, a second bounding element, preferably in the form of a toothed block, is provided at an inner side of the lumen of the second connector part at the lumen side and is adapted to cooperate with the corresponding first bounding element of the sealing pin in order to restrict a rotation of the sealing pin in at least one rotation direction.

In several embodiments, the second bounding element is formed as a plurality of ribs extending axially along the inside of the second connector part and/or as grooves in an inner surface of the second connector part.

In certain embodiments the first and the second bound elements form part of a rotation inhibition or limiting mechanism. Obviously, they provide just one example of such a mechanism comprised by some embodiments.

In several embodiments, the sealing pin is arranged in a common lumen formed by the lumens of the first and the second connector parts, with the sealing pin being movable by a relative movement of the first connector part relative to the second connector part between an open position in which the sealing pin enables a liquid flow through the common lumen and a closed position in which the sealing pin blocks the liquid flow through the common lumen.

In some embodiments, the third line is connected to, or comprises, the patient connector.

In certain embodiment, the patient connector is the second connector part or comprises the latter.

In several embodiments, the third line comprising a self-injecting and/or self-sealing mechanism for sealing the patient connector upon disconnection thereof.

In some embodiments, the sealing pin as described herein may be or may be part of the self-injecting and/or self-sealing mechanism.

In certain embodiments, the self-injecting and/or self-sealing mechanism is embodied as disclosed in WO 2021/043737 A1, the respective disclosure of which is incorporated herein by way of reference.

In several embodiments, the self-injecting and/or self-sealing mechanism is or comprises the first connector part having a lumen for conducting liquid; the first threaded portion that is arranged at an outer side of first connector part and that enables a releasable connection of the first connector part to the correspondingly matching second connector part; and a second threaded portion that is arranged at an inner side or at the lumen side of the first connector part and that enables a releasable connection of the first connector part to the correspondingly matching sealing pin, wherein the thread pitch of the first threaded portion preferably differs from the thread pitch of the second threaded portion.

In some embodiments, the patient connector comprises a manipulation limit or rim.

In several embodiments, the first line, the second line and/or the third line comprises an automatic line clamp, or the system provides an automatic line clamp for the first line, the second line and/or the third line. They may be controlled by a control device of the system.

In some embodiments, the system comprises at least one light emitting device for indicating the state of the automatic line clamp and/or manual line clamp, in particular, whether they are open or closed.

In several embodiments, the at least one optional light emitting device may be embodied as LED.

In certain embodiments, the at least one optional light emitting device may change its color in order to indicate the state of the clamp, e.g. red for closed, green for open. Other light signals, e.g. blinking, on/off, etc., may also be encompassed by the present invention.

In some embodiments, the first line comprises a frangible or the system provides a frangible provided for any one of the first, second and third line, in particular for the first line.

In order to break the frangible rather easily and, thereby, to allow a fluid flow, the first reusable unit may comprise a buckling aid. Alternatively, the tube dimensions of the lines are chosen in a way that a breaking edge of the frangible lies close to the edge or a through-opening, protrusion, edge or the like of the organizer featuring an edge in order to be broken by bending the tube over the edge.

In several embodiments, the first reusable unit comprises at least one fifth connection site for connecting a used or a pristine disinfection cap or sealing cap to the first reusable unit. The disinfection cap or sealing cap may be the closure element mentioned supra.

In some embodiments, the fifth connection site may be or comprise at least one fifth reception site.

In several embodiments, the system or the organizer comprises a disinfection cap or sealing cap.

In some embodiments, the system or the organizer comprises a control device that might have a microprocessor, a CPU, or other processing device. It may serve in order to process and/or control some or all of the automated functions of the system.

As automated functions may be understood, for example, some or all functions that do not involve human intervention or human provision. The control device may, thereby, automatically follow a program, algorithm or the like and is enabled to make decisions based on it in order to control and, if necessary, regulate the intended processes. The automated functions herein may include, e.g., prompting measurements, actuating automatic line clamps, automatic data acquisitions, data transmissions and the like.

In several embodiments, the control device is configured to control the actuation of automatic line clamps.

In some embodiments, the system comprises a flow sensor for liquid flow measurement and/or at least one, preferably electronic, scale for weight measurement, the flow sensor preferably being arranged on the first reusable unit.

In several embodiments, the control device is configured to control the actuation of the automatic line clamps in a time and/or volume controlled manner, preferably based on measured values from the flow sensor, the electronic scale and/or another sensor, e.g. as described herein.

In some embodiments, the control device is configured to keep the automatic line clamp at the third line and the automatic line clamp at the second line open for the, in particular initial, drain after patient connection until no flow - or no change in weight in case of alternatively using a scale - or no significant fluid flow or significant weight change is registered in a pre-determined time span and/or when the drainage of liquid from the peritoneal cavity of the patient is considered as complete.

As to the use of the term "initial drain": Starting a new APD treatment it is important to remove any fluid which may be located in the peritoneal cavity by means of the initial drain before the first fill cycle is executed. With the drain clamp pre-opened the drain may start spontaneously and immediately without any further action needed once the patient has been connected to the third line of the second disposable unit.

In several embodiments, the control device is configured to initiate draining, flushing, filling, dwelling and/or draining the first line, second line and/or third line.

In some embodiments, the electronic scale of the system is preferably provided enabled for the wireless communication with other parts of the organizer, in particular, with the control device.

In several embodiments, the control device is configured to prompt the removal of air present in the first line.

This may be effected by dialysis liquid flowing downwards from a liquid bag when connected to the first line and towards a drain bag connected to the second line while keeping one of the line clamps of the third line closed and by opening the line clamps of the first line and the second line, or keeping them open, for an adequate time.

In some embodiments, the control device is configured to allow the flow of fresh dialysis liquid along the first line and the third line into the peritoneal cavity for therapeutic reasons by keeping the automatic line clamp of the second line closed and by keeping the automatic line clamp of the first line and the automatic line clamp of the third line open until a prescribed liquid volume has been infused.

In several embodiments, the control device is configured to keep, during a prescribed therapeutic dwell time, all automatic line clamps closed.

In some embodiments, the control device is configured to maintain, during the draining of the dialysate from the peritoneal cavity, the automatic line clamp of the second line, herein also referred to as drain line, and the automatic line clamp of the third line open while monitoring the weight of one or more of the bags used until no further flow or no further change in weight (or no significant fluid flow or significant weight change) is registered in a certain time span and/or when the drainage of liquid for this cycle is considered to be complete.

In several embodiments, the control device is configured to determine the liquid volumes drained from the patient or infused into the patient either by timely integrating the values measured by the flow sensor coupled, e.g., to the measurement chamber or measurement area, or by monitoring an optional scale registering the differential weight change of fresh dialysis liquid and drained dialysate.

In these embodiments, the difference between drained (out-flown) and infused (in-flown) volume, or the change in total weight may represent the targeted ultrafiltration or even liquid reabsorption.

In some embodiments, the control device is configured to capture specific measurement values from a bidirectional fluid flow, i.e. during both the infusion of fresh dialysis liquid into the patient's peritoneal cavity and the drainage of dialysate from the peritoneal cavity, preferably using the same sensor for both measurements (in and out).

In several embodiments, the system comprises one or more sensors for the measurement of, e.g., glucose concentration, turbidity, temperature and/or conductivity, respectively. They are preferably coupled to the measurement chamber or measurement area or arranged to be coupled thereto. These sensors may serve for registering diagnostic data for therapy efficiency and improvement, preferably bidirectionally at the same measuring chamber or measurement area, i.e. by providing data from both the inflowing fresh dialysis liquid and the outflowing dialysate.

In some embodiments, the control device is configured to automatically screen the liquid drained from the peritoneal cavity by the turbidity sensor, preferably coupled to the measuring chamber or measuring area, for increased opacity in order to advantageously enhance patient safety. An increased opacity would indicate the presence of white blood cells or an increased concentration of proteins and hence point to a potential infection (Peritonitis).

In several embodiments, the control device is configured to calibrate the turbidity sensor during the inflow of the fresh dialysis fluid along the first line. This might help to improve the quality of the differential measurement signal, e.g., with the material of the connector and the liquid determining the base line.

In several embodiments, the control device is configured to, via the temperature sensor, monitor or continuously monitor the temperature of the fresh dialysis liquid inflowing along the first line and/or of the dialysate outflowing along the second line.

In these embodiments, the measured values can be used to correct all other temperature-dependent values. Further the temperature sensor can be used to monitor and control the temperature of the inflowing liquid and trigger the flow to stop should the measured temperature be outside the permitted safety limits. The temperature sensor coupled to the measuring chamber or measuring area can further measure the patient's core body temperature from the draining liquid and identify an elevated temperature or fever which could point towards a potential infection. Alone (e.g. in a bidirectional measurement) or combined with the results of the turbidity measurement this could hint towards a Peritonitis.

In several embodiments, the control device is configured to measure via the glucose sensor the respective glucose concentration of the fresh dialysis liquid, e.g., originating from a bag, a mixture of bags or on-site prepared, during inflow along the first line and/or of the dialysate during outflow along the third line, alternatively along the second or third line.

This value can then be compared to the glucose concentration prescribed and hence the adherence to the therapy prescription be monitored and confirmed or an alarm may be output in case of substantial discrepancies.

Also, any resulting glucose concentration from a mix of bags with different glucose content can be monitored or controlled and used for therapy relevant computation. This baseline measurement is in a second step compared to the glucose concentration in the outflowing dialysate. This way, further therapy-relevant information in order to determine therapy adequacy and water removal can be derived. The glucose sensor may be helpful in setting up automate routine tests of peritoneal membrane functionality and to allow for precision PD therapy.

In several embodiments, the control device is configured to measure, via the conductivity sensor, the respective conductivity of the fresh dialysis liquid, e.g., from a bag, a mixture of bags or on-site prepared liquid, in particular inside the first line or the third line.

This baseline measurement is in a second step compared to the conductivity of the outflowing dialysate. In this way, therapy-relevant information on treatment adequacy and peritoneal membrane performance can be derived, hence advantageously allowing for precision PD therapy.

In some embodiments, prior to the connection of the patient connector, the control device is configured to determine, via at least one of the sensors mentioned herein, e.g., the turbidity sensor, or via a combination of these sensors, when the initial priming of the second disposable unit and/or the third line (preferably comprising the patient line) is completed and when all air in the lines is removed.

In several embodiments, appropriated activation of the automatic line clamp of the second line will ensure air-free filling of the system and eventually termination of the filling procedure.

In some embodiments, the system comprises a barcode scanner, QR-Code reader and/or RFID sensor. These kinds of sensors may be able to read a set a of information, such as the type of a product, concentrations, an article number, a serial number, an expiry date, and/or the like.

This information from some form of, e.g., matrix (2D) barcode or RFID attached to or printed, e.g., on the side of the measuring chamber or measurement area may enable checks for therapy compliance and delivers product related information, e.g., for treatment documentation.

In several embodiments, the system comprises a pressure sensor for measuring the hydrostatic pressure within the patient's abdominal cavity, called the intraperitoneal pressure (IPP).

Such a pressure sensor may be positioned in a kind of shaft of the first reusable unit, next to the third connection site for the third line. It may then be able to measure the pressure within the third line very precise and without external interference, whereby this pressure corresponds to the IPP when the automatic line clamp of the third line is closed. Moreover, when closing the manual line clamp of the third line facing towards the patient and opening the corresponding line clamps of the first or the second line, the prevailing pressure within the connected liquid bags (for dialysis liquid or dialysate) may be determined, also very precise and without external interference. The opening and closing of the automatic line clamp may ensure that only pressure values exclusively originating from the patient's abdominal cavity are registered.

In some embodiments, the control device is configured to close, or keep closed, the automatic line clamp of the third line while measuring the pressure prevailing in the patient's abdominal cavity.

In several embodiments, the control device is configured to initiate measuring pressure values during the inflow, dwell or drainage of the dialysis liquid and to assess them. It may be kept open in order to measure changing pressure values during the inflow, dwell or drainage of the dialysis liquid, or when measuring those values.

In some embodiments, the control device is configured to automatically calibrate the pressure sensor, e.g., in periodic intervals. This may be effected by a procedure adjusting and fixing the dialysis liquid bag at a certain pre-defined height relative to the organizer, by closing the automatic line clamp of the second line and the automatic line clamp of the third line towards the patient, and by opening the automatic line clamp of the first line and/or the automatic line clamp of the third line to measure the hydrostatic reference pressure.

In several embodiments, prior to every treatment or frequently, one, several or all of the sensors may automatically be calibrated or checked, preferably in a separate procedure and/or during the filling process using standard CAPD or APD solution flowing along the sensors or the multi-sensor block, preferably by the control device. This is effected in order to advantageously define individual start or reference values, preferably based on fresh dialysis liquid bags, in a feasible way.

In some embodiments, the system comprises a multi-sensor block which in turn comprises some or all of the sensors of the system mentioned or described herein.

In several embodiments, the control device or another part of the system or the organizer comprises an external communication device, in particular, provided to act as a data gateway.

The safe and efficient communication between the organizer, other external devices, such as, e.g., the battery-powered digital scale, a heating device, a blood pressure monitor, etc.) and the external communication device may be established and managed, for example, via a dedicated application (short: App), e.g., as part of a holistic patient application or a device specific application.

When acting as a data gateway, the external communication device may provide all the state-of-the-art security and data protection measures modern smartphones, tablets or the like provide.

In some embodiments, the external communication device is used for user and/or patient identification and data assignment. This may preferably be realized via biometric recognition, e.g., a face-ID or finger-print sensor or via a personalized code, or a mix of these, using the proven appliances of modern external communication devices such as smartphones, tablets or the like already known from the prior art.

In several embodiments, the control device may be configured to initiate sending status information, e.g., clamp open or closed, the charging status of the battery, error codes, time stamps, etc., as well as the measurement results of some or all of the sensors of the system to the external communication device. This may in certain embodiments preferably be effected in short and/or regular intervals.

In some embodiments, the organizer supports wireless bi-directional communication, e.g., via Bluetooth, Wi-Fi or others, in order to be in or establish a signal communication with the independent external communication device, e.g., the patient's smartphone, tablet or the like.

When a signal communication or communication connection between two components is mentioned herein, this may be understood to mean a connection that exits during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

In several embodiments, the external communication device, which may itself be in signal communication with a secure data cloud, may receive and process some or all of the data and/or information mentioned herein. For this purpose, cloud computing (dedicated algorithms) may be used as known from the prior art.

In some embodiments, the external communication device may be configured in order to display data and/or information, e.g., results from its processing as described herein. Additionally or alternatively, it may be configured in order to send commands to open and close clamps and/or to initiate certain preparation, treatment, measurement or calibration procedures or status changes to the automated organizer. Via these processes the whole therapy or treatment may become advantageously digitalized, documented, stored, analyzed and/or controlled.

In several embodiments, the external communication device is configured to act as a remote control for the organizer and the wirelessly connected accessories and/or to features the central display of the system, i.e. where all potential user interface interactions take place.

In other embodiments, however, the organizer is able to function autonomously and is not necessarily dependent on the wireless connection to or presence of the external communication device. Any data measured or generated can be temporarily stored on the organizer, e.g., a suitable and provided storage device thereof, and is pushed to the external communication device and further onto the cloud whenever there is connection. This may in several embodiments apply for both ways.

In some embodiments, the system comprises a camera.

In several embodiments, the system comprises a heating device, in particular a heating plate, for heating and/or tempering the dialysis liquid source or bag. A power supply for this purpose may be provided and also be comprised by the system.

In some embodiments, the heating device comprises a communication device in order to be in signal communication with, e.g., the organizer and/or the external communication device or respective parts thereof.

In several embodiments, the heating device may be or comprise a box or container, particularly foldable.

In several embodiments, the heating device, or a box or container thereof, may comprise an insulating cover.

In several embodiments, the organizer displays a smooth shape, preferably shaped like a droplet, without protruding and/or sharp edges for enhanced comfort during use.

In certain embodiments, the control device is not configured to estimate particle concentration and/or leucocyte concentration, e.g., based on the measurements carried out by the sensors provided.

In some embodiments, the first reusable and/or the second disposable unit does not comprise an additional fluid line, or a fluid bypass line, for guiding fluid from the unit to sensor(s) provided therein or along the line/bypass line. This may apply, in particular for any type of sensor disclosed herein, optionally with the exception of the pressure sensor. The pressure sensor may be connected via a particular line/bypass line to the first reusable or the second disposable unit.

In certain embodiments, no substance for calibrating the sensors is provided or comprised by the system.

In some embodiments, urea is not measured and/or no percentage based on detected urea is calculated by the control device or any other element of the system.

In certain embodiments no disc with a dial and/or another element such as a connector, for example a so-called stay safe connector (which may be a part or a disc of a an organizer which is commercially distributed by Fresenius Medical Care, Germany, see also its disclosure in WO 2013/159935 A1, the respective disclosure of which is incorporated here by way of reference), that has to be inserted into or connected with the first reusable unit and/or the second disposable unit is needed for using the system and/or comprised thereof.

Some or all of the embodiments according to the present invention may comprise one, several or all of the advantages mentioned above and/or in the following.

An advantage of the present invention may be the suggestion of a simplified and more cost-effective disposable compared to the disposables of the prior art.

A further advantage of the present invention may be that due to the suggestion of the disposable with the sealing pin, the handling of the disposable is easier and safe, in particular with regard to the connection and disconnection of the patient before and after treatment. This may advantageously contribute to patient safety and hygiene. In general, the design of the pin connector advantageously enables low-contamination connection and disconnection to the third line of the second disposable unit.

The operation of the organizer of the system is advantageously easy and automated due to the integration of the external unit that may be already used in everyday life, e.g. the patient's smartphone. This may be a user friendly and easy-to-handle alternative to the treatment methods in the prior art.

The non-invasive diagnostic data acquisition on the suggested disposable provides extensive patient parameters and/or patient data without harming or bothering the patient. This may also contribute to patient safety and well-being on the one hand, while enabling a precise peritoneal dialysis treatment on the other

Further advantageously, CAPD (PD) therapy may be completely or at least largely digitized via the system.

The present invention may further advantageously be expandable to a cycler for APD therapy without much effort. This makes the system according to the present invention flexible in use, i.e. it may be used in a variety of ways.

Measuring parameter values by means of the sensors provided at or on the first reusable unit may contribute to achieving quickly and even on-site results. Specimen do not have to be sent to the lab.

A further advantage of the present invention may be that due to the small and compact design of the system it may be suitable to be used during travelling and as well as an easily storable modular system in conjunction with an external scale and/or a heater.

All advantages achievable with the method according to the present invention can be in certain embodiments achieved undiminished by the apparatuses according to the present invention and vice versa.

In the following, the present invention is described based on preferred embodiments thereof with reference to accompanying drawing. However, the present invention is not to be limited to these embodiments. In the figures, in which same reference numerals denote identical or similar elements, values and so on, the following applies:
- **Fig. 1**: shows a first reusable unit of an organizer of a system according to the present invention in an exemplary embodiment;
- **Fig. 2**: shows a second disposable unit of an organizer of a system according to the present invention in an exemplary embodiment;
- **Fig. 3**: shows a second disposable unit of an organizer of a system according to the present invention in a further exemplary embodiment;
- **Fig. 4**: shows the first reusable unit and the second disposable unit of Fig. 1 and Fig. 2 in a connected state;
- **Fig. 5**: shows the organizer of the preceding figure in a sectional view from the side; and
- **Fig. 6**: shows a system according to the present invention in a highly simplified representation.

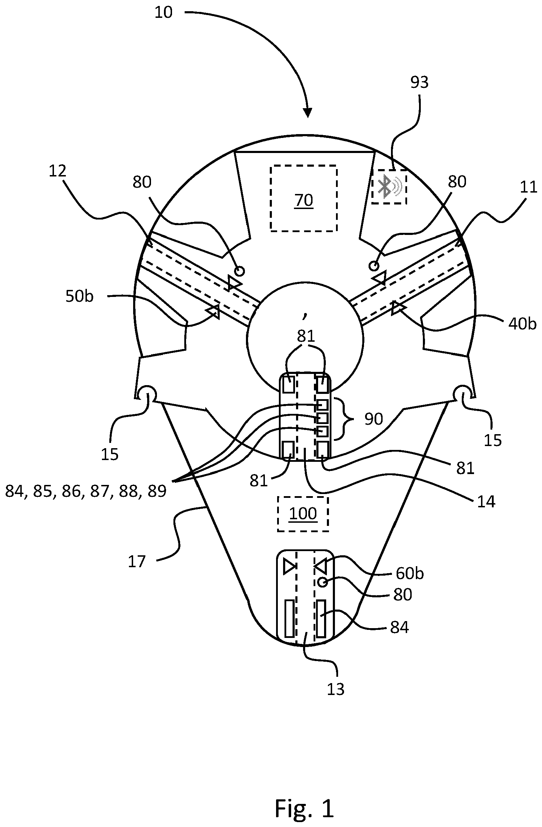

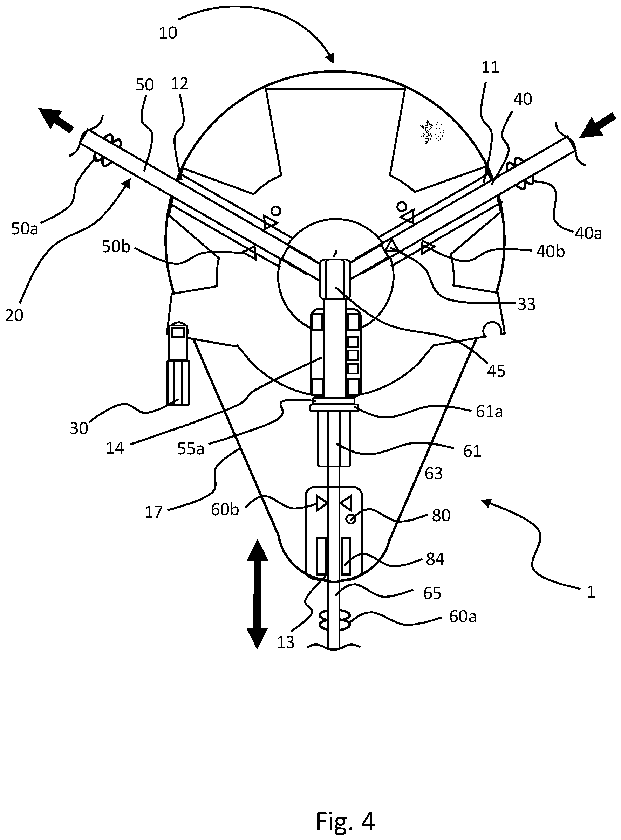

**Fig. 1** shows a first reusable unit 10 of an organizer 1 (see Fig. 4) of a system according to the present invention in an exemplary embodiment.

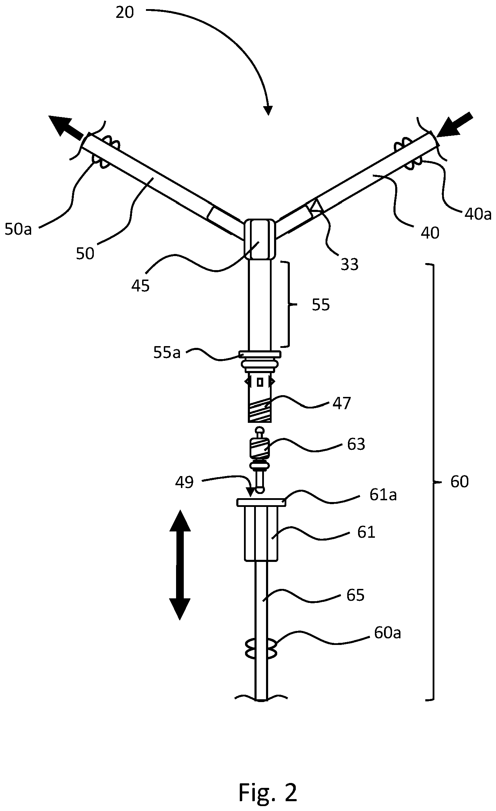

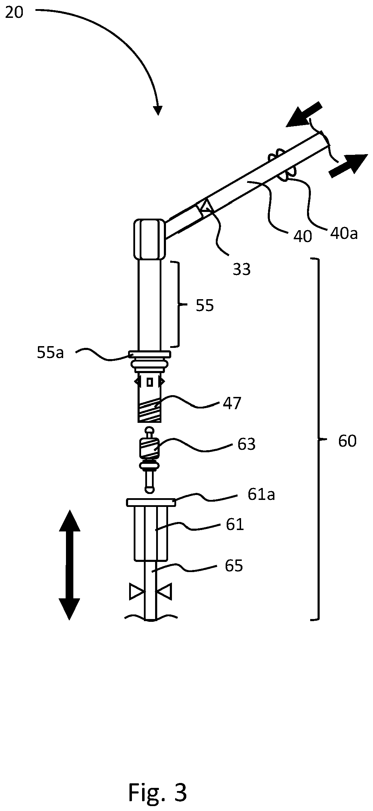

The first reusable unit 10 may be understood as a housing 17 or a tray, reception or support for receiving a second disposable unit 20 (see Fig. 2 or Fig. 3).

The housing 17 is preferably made of a hard, disinfectable material or comprises such.

As stated supra, the first reusable unit 10 may be provided in order to be connected to the second disposable unit 20 (see Fig. 2 and Fig. 4).

For this purpose, in the example of Fig. 1, the first reusable unit 10 comprises connection sites, in the example of Fig. 1 there are five connection sites 11, 12, 13, 14, 15 (fewer or more connection sites are contemplated as well), here exemplarily designed as grooves or comprising grooves.

In order to avoid repetitions, reference is made to the description with regard to the following figures at this point.

In the example of Fig. 1, the first connection site 11, the second connection site 12 and/or the third connection site 13 comprises an optional automatic line clamp 40b, 50b, 60b, respectively, the purpose of which is explained with regard to Fig. 4.

An optional light emitting device 80, e.g., an LED, is positioned adjacent to some or all of the automatic line clamps 40b, 50b, 60b. The light emitting devices 80 are optionally provided for indicating the state of the automatic line clamp 40b, 50b, 60b. This will also be explained in more detail with regard to Fig. 4.

The optional fourth connection site 14 is provided and/or suitable to receive the third line 60, preferably the measurement chamber 55 or area thereof, that may be connected to a patient line 65 (Fig. 2 and Fig. 3) while treating a patient P (not shown in Fig. 1). The first, second and third lines 40, 50, 60 as well as the Y-piece 45 are described in more detail with regard to Fig. 2.

The optional fifth connection site 15 is provided and/or suitable to receive a disinfection cap 30 (see Fig. 4) of the third line 60, in particular a used or a pristine disinfection cap 30 of the patient connector 61. This is also described in more detail with regard to Fig. 4.

The organizer 1, in particular the first reusable unit 10 thereof, or any other part of the system may further comprise a control device 100 that may in turn comprise a microprocessor or CPU. The control device 100 may be configured or programmed to process and/or control some or all of the automated functions, measurements, data acquisitions and/or data transmissions. The control device is preferably housed within the housing 17.

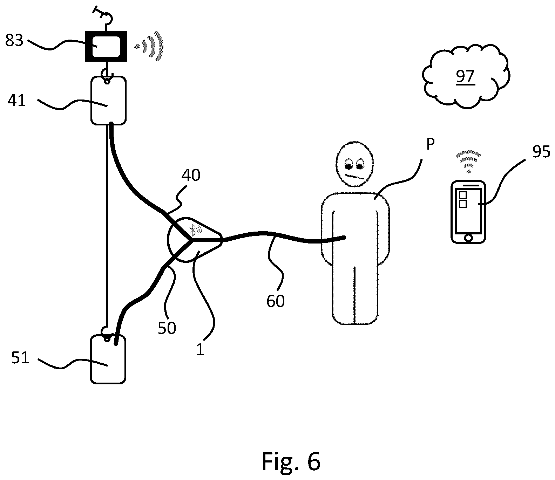

The control device 100 may further be configured for prompting the actuation of the automatic line clamps 40b, 50b, 60b. This may in some embodiments be effected in a time controlled and/or volume controlled manner, preferably based on measured values from various sensors, for example, an optional flow sensor 81. Other types of sensors may be, independently from each other, in addition or instead, also comprised by the present invention, e.g. as part of the system in general or as part of the first reusable unit 10, e.g., an electronic scale 83 (not shown in Fig. 1, please see Fig. 6), pressure sensor 84, turbidity sensor 85, barcode scanner 86, QR-code scanner, RFID scanner, temperature sensor 87, glucose sensor 88, conductivity sensor 89.

At least two of such sensors may in some embodiments form a multi-sensor block 90, e.g., with the sensors 84, 85, 86, 87, 88, 89 arranged in an array, preferably all reaching, i.e. having access, to an optional measurement chamber 55 of, or in contact with or near, the Y-piece 45 (please see the description with regard to Fig. 2 and Fig. 4 for further details).

A communication device 93 may also be comprised by the system, e.g. by the first reusable unit 10, e.g. within its housing 17. The purpose of the communication device 93 is described in more detail with regard to Fig. 6.

The control device 100 may be configured to initiate treatment steps when using the system according to the present invention, namely, e.g., draining, flushing, filling, dwelling and/or draining the first, second and/or third lines 40, 50, 60.

As a power supply, e.g. for the control device 100, the automatic line clamps 40b, 50b, 60b, the sensors 81, 84, 85, 86, 87, 88, 89, the communication device 93 and/or optional other devices of the organizer 1, the first reusable unit 10 may comprise at least one battery or rechargeable battery 70 including the connections, i.e. for charging, necessary for its proper use.

**Fig. 2** shows a second disposable unit 20 of an organizer 1 (see Fig. 4) of a system in an exemplary embodiment.

In the example of Fig. 2 the second disposable unit 20 comprises a first line 40 for guiding fresh dialysis liquid from a dialysis liquid source or bag (not shown in Fig. 2, please see Fig. 6) towards, e.g., a Y-piece 45 or connector, preferably also comprised by the second disposable unit 20.

The second disposable unit 20 further comprises a second line 50 for guiding used dialysis liquid, herein also referred to as dialysate, away from the Y-piece 45 or connector towards a drain or drain bag 51 (not shown in Fig. 2, please see Fig.6).

The Y-piece 45 is in fluid communication or connected to a third line 60 that is also comprised by the second disposable unit 20. The third line 60 is suitable and/or provided for, directly or indirectly, guiding fresh dialysis liquid from or through the organizer 1, in particular away from the Y-piece 45, towards a patient P (not shown in Fig. 2, please see Fig. 6) and dialysate away from the patient P to or through the organizer 1, in particular towards the Y-piece 45.

In the embodiment of Fig. 2, the part of the third line 60 the end of which may be connected to the Y-piece 45 may also be referred to as measurement chamber 55 or measurement area, whereas any other portion of the Y-piece 45 or the third line 60 may do as well in certain embodiments.

The measurement chamber 55 or area may be an integrated cuvette-type coupling area for non-invasive sensors, e.g. as arranged in the multi-sensor block 90 as described herein.

The measurement chamber may display a manipulation limit or rim 55a.

The Y-piece 45 may comprise at its end facing the third line 60 a first connector part 47, preferably a first screw fitting, for connecting it to a corresponding second connector part 49, here a corresponding inner thread, of a patient connector 61, preferably embodied as a receiving section, preferably concentrically surrounding the lumen.

In the embodiment of Fig. 2 the second disposable unit 20 optionally comprises a self-sealing and/or self-injecting mechanism, realized by a sealing pin 63. This part of the second disposable unit 20 is shown in an exploded view for better understanding.

The sealing pin 63 is designed to fit between the Y-piece 45 and the patient connector 61 positioned at the end of the third line 60 facing the Y-piece 45 or into or between the connector parts 47, 49 thereof. The patient connector 61 may display a manipulation limit or rim 61a, too.

The sealing pin 63 optionally comprises an elongated body with a projecting thread that creates a lumen for enabling a liquid to be conducted within the first connector part 47 and the second connector part 49 when these parts are connected.

The projecting thread of the sealing pin 63 may be connected to a threaded portion at the inner side of the first connector part 47. At the first connector part 47, the thread pitch of the inner thread for connecting the sealing pin 63 and the thread pitch of the outer thread for connecting the third line 60 preferably differ from each other.

The sealing pin 63 is preferably arranged within the lumen of the first connector part 47 so that liquid can flow through and/or around the sealing pin 63 through the first connector part. It may, furthermore, comprise a first bounding element, preferably in the form of a toothed block, that restricts a rotation of the sealing pin 63 to one direction when being connected or in form-fit engagement to a second bounding element within the lumen of the second connector part 49. Such a second bounding element may be formed as a plurality of ribs extending axially along the inner side of the wall of the lumen of the second connector part 49. At the end opposite the first bounding element, the sealing pin 63 preferably has a fixing element, by which fixing element the sealing pin 63 can be releasably or non-releasably fixed to a closure element, preferably via a latched connection and/or via an undercut.

The sealing pin 63, when arranged in the lumen of the second connector part 49, enables liquid to flow through or along the sealing pin 63 and the lumen of the second connector part 49.

The sealing pin 63 is arranged in the common lumen formed by the lumina of the first and second connector parts 47, 49, with the sealing pin 63 being movable by a relative movement of the first connector part 47 to the second connector part 49 between an open position in which the sealing pin 63 enables a liquid flow through the common lumen and a closed position in which the sealing pin 63 blocks the liquid flow through the common lumen. In other words, when disconnecting the patient connector 61 from the Y-piece, the sealing pin 63 remains in the second connector part 49 of the patient connector 61 in order to seal it against leakage and/or, more important, the hygienically safe protection against possible intrusion of microorganisms.

Each of the first, second and/or third lines 40, 50, 60 optionally comprise a manual line clamp 40a, 50a, 60a, independently from each other.

A frangible 33 that has to be broken, or any other element that has to be manipulated before the first use of the second disposable unit 20, may optionally be provided in the first line 40.

This arrangement may be advantageously used with continuous ambulatory peritoneal dialysis (CAPD) treatments.

**Fig. 3** shows a second disposable unit 20 of an organizer 1 (not shown in Fig. 3) of a system in a further exemplary embodiment.

Reference is made to the reference numerals and the description with regard to Fig. 2. Only the differences to the preceding figure will be described at this point.

In the example of Fig. 3, the first line 40 may be provided and/or suitable in order to guide both fresh dialysis liquid from a dialysis liquid source 41 (see Fig. 6) towards the Y-piece 45 and dialysate away from the Y-piece towards a drain 51 (see Fig. 6) or an optional cycler.

This arrangement may be advantageously used with ambulatory peritoneal dialysis (APD) treatments. Moreover, when a cycler is connected to the first line 40 of this arrangement, it could be used to perform a CCPD.

**Fig. 4** shows the first reusable unit 10 and the second disposable unit 20 of the Fig. 1 and Fig. 2 in a connected state, forming an embodiment of the organizer 1.

Reference is made to the reference numerals and descriptions with regard to the preceding figures. Some of the reference numerals were omitted in order to facilitate the overview.

The first line 40 is received in the first connection site 11 of the first reusable unit 10, the second line 50 in the second connection site 12, and the third line 60 in the third connection site 13.

The Y-piece 45 is connected to the third line 60, i.e. the measurement chamber 55 thereof, in the fourth connection site 14, preferably so that the measurement chamber 55 or area may get in contact with the provided sensors as described with regard to Fig. 1.

The automatic line clamps 40b, 50b and 60b now have effect on the corresponding first, second and third lines 40, 50, 60 in the connection sites 11, 12, 13. Therefore, the flow within the first, second and third lines 40, 50, 60 may be controlled or regulated by the control device 100.

The following examples may be representative for possible combinations of open/closed clamps. The examples do not claim to be complete.

For example, after the optional frangible 33 has been broken, the automatic line clamp 40b at the first line 40 and the automatic line clamp 60b at the third line 60 may be opened while simultaneously the automatic line clamp 50b at the second line 50 is kept close in order to fill the peritoneal cavity of the patient P (in Fig. 4 not shown) with fresh dialysis liquid out of a liquid source 41 (see Fig. 6).

With the automatic line clamp 60b kept close, the intraperitoneal pressure (IPP) may be measured, e.g. by the pressure sensor 84 at the third (patient) line 60.

In order to empty the peritoneal cavity, e.g. after treatment or in order to exchange the used dialysis liquid, i.e. the dialysate, for fresh dialysis liquid, the automatic line clamp 60b at the third line 60 and the automatic line clamp 50b at the second line 50 may be opened for the initial and immediate drain of the peritoneal cavity of the patient P (not shown in Fig. 4) until the drainage is completed. The completion of the drainage may exemplarily be monitored by an electronic scale 83 (see Fig. 6).

The light emitting devices 80 may be provided in order to indicate the state of the automatic line clamps 40b, 50b, 60b, i.e. whether they are open or closed. For example, they may change their color for this purpose, e.g. red for closed, green for open. Other light signals, e.g. blinking, on/off, etc., may also be encompassed by the present invention.

A used or pristine disinfection cap 30 which is not needed during the treatment of the patient P may be received by the fifth connection site 15 of the first reusable unit 10.

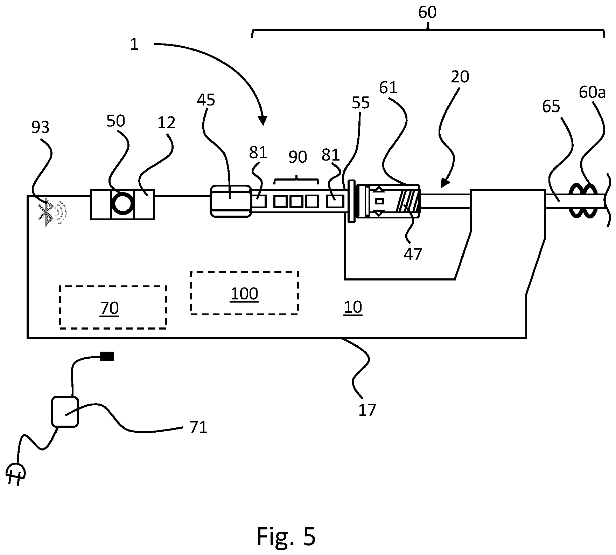

**Fig. 5** shows the organizer 1 of the preceding figures in a sectional view from the side.

Reference is made to the reference numerals and descriptions with regard to the preceding figures.

In Fig. 5 the design of the second connection site 12 as a groove with the connected second line 50 received in it can be seen as an example for the reception of all three lines 40, 50, 60.

The control device 100 is covered within the first reusable unit 10, in particular in the housing 17 thereof, also the battery or rechargeable battery 70 of the organizer 1. A charging device 71 is shown next to the organizer 1. The charging device 71 is symbolic of all charging devices of the prior art, e.g. inductive or the like
The flow sensor 81 and the sensors 84, 85, 86, 87, 88, 89 (not explicitly denoted in Fig. 5) of the optional multi-sensor block 90 lie adjacent to the measurement chamber 55 of the third line 60 in order to acquire the data as described herein.

**Fig. 6** shows a system according to the present invention in a highly simplified representation.

Reference is made to the reference numerals and descriptions with regard to the preceding figures.

The system according to the present invention comprises an organizer 1 for medical treatment, in the example of Fig. 6 for a dialysis treatment, in particular for a peritoneal dialysis treatment. This kind of treatment is represented by the third line 60 of a second disposable unit 20 (see Fig. 2) leading liquid to and from the peritoneal cavity of a patient P.

Besides the second disposable unit 20, in the example of Fig. 6 highly simplified represented only by the three lines 40, 50, 60, the organizer 1 further comprises the first reusable unit 10, exemplarily designed as described with regard to the preceding figures, although only shown very schematically in Fig. 6 by its external outline.

The first line 40 guides fresh dialysis liquid out of a dialysis liquid source or bag 41, to or through the organizer 1 via the third line 60 towards the patient, preferably without using any conveying devices, i.e. only driven by gravity.

After a determined time span, e.g. several hours, used dialysis liquid, i.e. dialysate, is guided out of the peritoneal cavity of the patient P via the third line 60 to or through the organizer 1 and is then guided by the second line 50 to a drain or drain bag 51, again preferably driven by gravity.

The flows through the lines 40, 50, 60 may be controlled by a control device 100 (see Fig. 1) which is configured to control the automatic line clamps 40b, 50b, 60b (see Fig. 1) e.g. as described elsewhere herein.

The system may comprise at least one electronic scale 83 for balancing the treatment fluids, preferably battery powered. In the example of Fig. 6 there is arranged one electronic scale 83 weighing both the dialysis liquid source, here implemented as a bag 41, and the drain, here also implemented as a bag 51, e.g. in order to determine the completion of the treatment or to monitor its execution. There is only one scale 83 in the example of Fig. 6. Any other number of scales is also encompassed in the present invention.

In order to fulfil its numerous purposes, the organizer 1 may be battery powered, e.g. by a rechargeable
battery/accumulator, e.g. as described with regard to Fig. 5.

Moreover, the organizer 1 may support wireless bi-directional communication, e.g. via Bluetooth, Wi-Fi or the like, with an independent external communication device 95, in the example of Fig. 6 represented by a smartphone, tablet or the like.

In some embodiments of the organizer 1, the external communication device 95 may act as data gateway. The safe and efficient communication between the organizer 1, the other external devices, e.g., the battery-powered digital scale 83, heating device, blood pressure monitor, etc., and the external communication device 95 is established and managed via a dedicated application (in short: App), e.g., as part of a holistic patient app or a device specific application. The external communication device 95 then acts as data gateway and provides all the state-of-the-art security and data protection measures modern smartphones or tablets provide.

User and/or patient identification and data assignment may preferably also be provided by the external communication device 95, e.g. realized via biometric recognition, e.g., Face-ID or finger-print sensor or via a personalized code, or a mix of these, using the proven appliances of modern external communication devices from the prior art.

The control device 100 of the organizer 1 may in some embodiments be configured in order to initiate sending status information (e.g., clamp open or closed, charging status battery, error codes, time stamps, etc.) and the measurement results of some or all of the sensors 81, 83, 84, 85, 86, 87, 88, 89, preferably in short and/or regular intervals, to the external communication device 95.

The external communication device 95 may in turn be in connection with a secure data cloud 97 itself, receiving and processing these data, potentially also with the aid of cloud computing (dedicated algorithms), displaying the results and sending commands to open and close clamps or initiating certain preparation, treatment, measurement or calibration procedures or status changes to the organizer 1. Via these processes the whole PD therapy or treatment gets digitalized, documented, stored, analysed and controlled.

The present invention is not limited to the embodiments described herein. These are for illustrative purposes only.

### Reference Numerals

- 1: organizer
- 10: first reusable unit
- 11: first connection site
- 12: second connection site
- 13: third connection site
- 14: fourth connection site
- 15: fifth connection site
- 17: housing
- 20: second disposable unit
- 30: disinfection caps
- 33: frangible
- 40: first line
- 40a: manual line clamp
- 40b: automatic line clamp
- 41: dialysis liquid source or bag
- 45: Y-piece
- 47: first connector part
- 49: second connector part
- 50: second line
- 50a: manual line clamp
- 50b: automatic line clamp
- 51: drain; drain bag
- 55: measurement chamber
- 55a: manipulation limit or rim
- 60: third line
- 60a: manual line clamp
- 60b: automatic line clamp
- 61: patient connector
- 61a: manipulation limit or rim
- 63: sealing pin
- 65: patient line
- 70: battery; rechargeable battery
- 71: charging device
- 80: light emitting device; LED
- 81: flow sensor
- 83: (electronic) scale
- 84: pressure sensor
- 85: turbidity sensor
- 86: barcode scanner; QR-code scanner; RFID scanner
- 87: temperature sensor
- 88: glucose sensor
- 89: conductivity sensor
- 90: multi-sensor block
- 93: communication device
- 95: external communication device
- 97: cloud
- 100: control device

## Claims

1. A system comprising an organizer (1) for medical treatment, preferably for a dialysis treatment of a patient (P), in particular for a peritoneal dialysis treatment, the organizer (1) comprising
- a first reusable unit (10), comprising at least one first connection site (11) for connecting it to a second disposable unit (20), the second disposable unit (20) being or comprising lines.

2. The system according to claim 1, wherein the first reusable unit (10) comprises or is connected to the second disposable unit (20).

3. The system according to any one of the preceding claims, the second disposable unit (20) comprising a first line (40) for guiding fresh dialysis liquid from a dialysis liquid source or bag (41), a second line (50) for guiding used dialysis liquid to a drain or drain bag (51), both lines (40, 50) being in fluid connection or connected to each other, preferably via a Y-piece (45) or another line section, the Y-piece (45) or other line section being in fluid connection or connected to a third line (60) for guiding fresh dialysis liquid towards and used dialysis liquid away from the patient (P).

4. The system according to any one of the preceding claims, the second disposable unit (20) comprising a first line (40) for guiding fresh dialysis liquid from a dialysis liquid source or bag (41) towards and for guiding used dialysis liquid to a drain or drain bag (51) away from the organizer (1), and a third line (60) in fluid connection or connected to the first line (40), the third line (60) for guiding fresh dialysis liquid towards and used dialysis liquid away from the patient (P).

5. The system according to any one of claim 3 to claim 4, the Y-piece (45) or the third line (60) comprising or being embodied as a measurement chamber (55) or measurement area.

6. The system according to any one of claim 3 to claim 5, the third line (60) comprising a screw fitting for connecting the third line (60) with a sealing pin (63) also comprised.

7. The system according to any one of claim 3 to claim 6, the third line (60) comprising a self-injecting and/or self-sealing mechanism for sealing the patient connector (61) upon disconnection thereof.

8. The system according to the preceding claim, the self-injecting and/or self-sealing mechanism being or comprising a first connector part (47) having: a lumen for conducting a liquid; a first threaded portion that is arranged at an outer side of the first connector part (47) and that enables a releasable connection of the first connector part (47) to a correspondingly matching second connector part (49); and a second threaded portion that is arranged at an inner side of the first connector part (47) at the lumen side and that enables a releasable connection of the first connector part (47) to a correspondingly matching sealing pin (63), wherein the thread pitch of the first threaded portion differs from the thread pitch of the second threaded portion.

9. The system according to any one of preceding claims, wherein the first line (40) comprises a frangible (33), or wherein a frangible (33) is provided for the first line (40).

10. The system according to any one of the preceding claims, wherein the system or the organizer (1) comprises a control device (100) to process and/or control some or all of the automated functions, such as prompting measurements, actuating automatic line clamps (40b, 50b, 60b), data acquisitions and/or data transmissions and the like.

11. The system according to any one of the preceding claims, comprising a flow sensor (81) or scale (83) for liquid flow or weight measurement, preferably arranged on the first reusable unit (10).

12. The system according to any one of claim 10 to claim 11, wherein the control device (100) is configured to control the actuation of the automatic line clamps (40b, 50b, 60b) in a time and/or volume controlled manner, preferably based on measured values from the flow sensor (81), the electronic scale (83) and/or another sensor (85) of the system.

13. The system according to any one of claim 10 to claim 12, wherein the control device (100) is configured to initiate draining, flushing, filling, dwelling and/or draining the first line (40), second line (50) and/or third line (60).

14. Method for preparing an organizer (1) before treating a patient by dialysis, encompassing the steps:
- providing a system according to any one of claims 9 to 13;
- breaking the frangible (33).

15. A second disposable unit (20) as described in one of claims 1 to 13.
